(19) Europäisches Patentamt
European Patent Office
Office européen des brevets

(11) EP 1 621 180 A1

(12) **DEMANDE DE BREVET EUROPEEN**

(43) Date de publication:
**01.02.2006 Bulletin 2006/05**

(51) Int Cl.:
***A61K 8/18*** *(2006.01)*

(21) Numéro de dépôt: **05291946.1**

(22) Date de dépôt: **18.11.1999**

(84) Etats contractants désignés:
**AT BE CH CY DE DK ES FI FR GB GR IE IT LI LU MC NL PT SE**

(30) Priorité: **26.11.1998 FR 9814907**

(62) Numéro(s) de document de la (des) demande(s) initiale(s) en application de l'article 76 CBE:
**99956076.6 / 1 051 145**

(71) Demandeur: **L'OREAL S.A.**
**75008 Paris Cedex (FR)**

(72) Inventeurs:
- **Samain, Henri**
**91570 Bievres (FR)**
- **Dupuis, Christine**
**75018 Paris (FR)**

(74) Mandataire: **Bourdeau, Françoise**
**L'OREAL - D.I.P.I.**
**25-29 Quai Aulagnier**
**92600 Asnières (FR)**

Remarques:
Cette demande a été déposée le 30-09-2005 comme demande divisionnaire de la demande mentionnée sous le code INID 62.

(54) **Composition de coiffage comprenant un polymère aux caractéristiques particulières et un polymère non ionique**

(57) L'invention a pour objet une composition de coiffage comprenant, dans un milieu cosmétiquement acceptable:

(1) au moins un polymère (A) choisi de telle sorte qu'un film obtenu par séchage d'un mélange de ce polymère (A) avec de l'éthanol ou de l'eau, à température ambiante et à un taux d'humidité relative de 50 %, présente un profil mécanique défini par au moins:

(i) un taux d'allongement à la rupture ($\varepsilon_r$) supérieur ou égal à 300 %;
(ii) une recouvrance à 300 secondes ($R_{300}$) supérieure ou égale à 45 %; et
(iii) lorsque la recouvrance à 300 secondes est comprise entre 45 et 60 %, alors l'allongement est inférieur à 1300 %;

(2) au moins un polymère (B) filmogène, non ionique, différent du polymère (A).

Elle vise également un procédé de mise en forme ou de maintien des cheveux à l'aide de cette composition ainsi que son utilisation pour la formulation de produits de coiffage tels que les laques, les sprays ou les mousses, en vue d'obtenir le maintien ou la mise en forme de la coiffure.

EP 1 621 180 A1

## Description

L'invention a pour objet une composition de coiffage comprenant, dans un milieu cosmétiquement acceptable, au moins un polymère (A) aux caractéristiques particulières et au moins un polymère (B) filmogène et non ionique. Elle vise également un procédé de mise en forme ou de maintien des cheveux à l'aide de cette composition ainsi que son utilisation pour la formulation de produits de coiffage tels que les laques, les sprays ou les mousses, en vue d'obtenir le maintien ou la mise en forme de la coiffure.

Les produits capillaires pour la fixation des cheveux les plus répandus sur le marché de la cosmétique sont des compositions à pulvériser en aérosol ou en flacon pompe tels que les laques, les sprays ou les mousses, essentiellement constituées d'une solution le plus souvent alcoolique ou hydroalcoolique et d'un polymère filmogène soluble dans l'eau ou dans l'alcool, en mélange avec divers adjuvants cosmétiques.

Toutefois, ces formulations capillaires telles que les mousses, gels et surtout les sprays et les laques aérosols destinées à maintenir la forme de la coiffure ne permettent encore pas à la coiffure de résister de manière satisfaisante aux différents mouvements naturels de la vie comme la marche, les mouvements de tête ou les coups de vent.

Les polymères utilisés pour la formulation de ces produits capillaires sont des polymères filmogènes anioniques, amphotères ou non ioniques, qui conduisent à la formation de films possédant un caractère plus ou moins dur et cassant.

Lorsque le polymère est trop cassant, le pourcentage d'allongement à la rupture mesuré sur le film est faible, c'est-à-dire en général inférieur à 2 % et la tenue de la coiffure n'est pas assurée dans le temps.

Pour remédier à ce problème, on a déjà mélangé ces polymères avec des plastifiants et obtenu des revêtements plus souples et non friables. Toutefois, ces films sont déformables et plastiques, c'est-à-dire qu'après déformation, ils ne récupèrent que très peu de leur forme initiale. Si la tenue de la coiffure est améliorée, elle n'est pas encore satisfaisante puisque la forme de la coiffure évolue dans le temps.

Des résultats plus satisfaisants en terme de tenue ont été obtenus avec des compositions comprenant une association de polymères filmogènes, tels que par exemple un polymère cellulosique et un polymère acrylique. Toutefois, ces compositions ne donnent encore pas entièrement satisfaction, dans la mesure où les cheveux perdent certaines de leurs propriétés cosmétiques naturelles.

On recherche donc des compositions cosmétiques pour le maintien et/ou la fixation de la coiffure qui procurent à la chevelure, outre une fixation durable, de bonnes propriétés cosmétiques, notamment un bon démêlage, de la douceur et un aspect agréable.

De manière surprenante et inattendue, la Demanderesse a découvert qu'il était possible de remédier aux problèmes techniques évoqués ci-dessus en utilisant certaines associations spécifiques de polymères.

L'invention a pour objet composition de coiffage comprenant, dans un milieu cosmétiquement acceptable:

(1) au moins un polymère (A) choisi de telle sorte qu'un film obtenu par séchage d'un mélange de ce polymère (A) avec de l'éthanol ou de l'eau, à température ambiante et à un taux d'humidité relative de 50 %, présente un profil mécanique défini par au moins:

(i) un taux d'allongement à la rupture ($\varepsilon_r$) supérieur ou égal à 300 %;
(ii) une recouvrance à 300 secondes ($R_{300}$) supérieure ou égale à 45 %; et
(iii) lorsque la recouvrance à 300 secondes est comprise entre 45 et 60 %, alors l'allongement est inférieur à 1300 %;

(2) au moins un polymère (B) filmogène, non ionique, différent du polymère (A) et choisi parmi

- les polyalkyloxazolines;
- les homopolymères d'acétate de vinyle;
- les copolymères d'acétate de vinyle et d'ester acrylique;
- les copolymères d'acétate de vinyle et d'éthylène;
- les copolymères d'acétate de vinyle et d'ester maléïque;
- les copolymères de polyéthylène et d'anhydride maléïque;
- les homopolymères d'acrylates d'alkyle et les homopolymères de méthacrylates d'alkyle;
- les copolymères d'esters acryliques tels que par exemple les copolymères d'acrylates d'alkyle et de méthacrylates d'alkyle ;
- les copolymères d'acrylonitrile et d'un monomère non ionique choisi parmi le butadiène et les (méth)acrylates d'alkyle,
- les copolymères d'acrylate d'alkyle et d'uréthanne,
- les gommes de guar,
- les polymères dérivés d'hydroxystyrène,

- les homo ou copolymères de vinyllactames.

Un autre objet de la présente invention concerne un procédé de mise en forme ou de maintien de la coiffure comprenant la mise en oeuvre de cette composition.

Encore un autre objet de la présente invention concerne l'utilisation de cette composition pour la fabrication de compositions cosmétiques capillaires, en vue d'obtenir un maintien ou une mise en forme de la coiffure.

Les polymères (A) particulièrement visés par la présente invention sont ceux distribués par Goodrich sous l'appellation Avalure AC 315® et V29®.

Au sens de la présente invention, on entend par film obtenu par séchage à température ambiante (22 $\pm$ 2°C) et à un taux d'humidité relative de 50 % $\pm$ 5%, le film obtenu dans ces conditions à partir d'un mélange à 6 % de matière active (m.a.) de polymère A avec de l'éthanol ou de l'eau, la quantité de mélange étant adaptée pour obtenir dans une matrice en téflon, un film d'épaisseur de 500 $\pm$ 50 $\mu$m. Le séchage est poursuivi jusqu'à ce que le poids du film n'évolue plus, ce qui représente environ 12 jours. Les polymères A solubles ou partiellement solubles dans l'éthanol sont testés dans l'éthanol. Les autre polymères sont testés dans l'eau sous forme soluble ou dispersée.

Au sens de la présente invention, le taux d'élongation à la rupture et le taux de recouvrance sont évalués aux moyens des essais décrits ci-après.

Pour effectuer les essais de traction, le film est découpé en éprouvettes de forme rectangulaire, de longueur 80 mm et de largeur 15 mm.

Les essais sont réalisés sur un appareil commercialisé sous l'appellation Lloyd ou commercialisé sous l'appellation Zwick dans les mêmes conditions de températures et d'humidité que pour le séchage, c'est-à-dire une température de 22 $\pm$ 2 °C et un taux d'humidité relative de 50 $\pm$ 5 %.

Les éprouvettes sont étirées à la vitesse de 20mm/mn et la distance entre les mors est de 50 $\pm$ 1 mm.

Pour déterminer la recouvrance instantanée ($R_i$), on procède comme suit:

- on étire l'éprouvette de 150 % ($\varepsilon_{max}$) c'est-à-dire 1,5 fois sa longueur initiale ($l_0$)
- on relâche la contrainte en imposant une vitesse de retour égale à la vitesse de traction, soit 20mm/mn et on mesure l'allongement de l'éprouvette en pourcentage , après retour à charge nulle ($\varepsilon_i$).

La recouvrance instantanée en % ($R_i$) est donnée par la formule ci-après:

$$R_i = ((\varepsilon_{max} - \varepsilon_i)/ \varepsilon_{max}) \times 100$$

Pour déterminer la recouvrance à 300 secondes, on maintient à contrainte nulle pendant 300 secondes supplémentaires, l'éprouvette ayant subi les opérations précédentes, et on mesure son taux d'allongement en pourcentage ($\varepsilon_{300}$).

La recouvrance en % à 300 secondes ($R_{300}$) est donnée par la formule ci-après:

$$R_{300} = ((\varepsilon_{max} - \varepsilon_{300})/ \varepsilon_{max}) \times 100$$

Les polymères (B) filmogènes non ioniques utilisables selon la présente invention sont choisis de préférence parmi :

- les homopolymères de vinylpyrrolidone ou de vinylcaprolactame;
- les copolymères de vinylpyrrolidone et d'acétate de vinyle ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;

- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.
- les gommes de guar non ioniques chimiquement modifiées ou non modifiées.

Les gommes de guar non ioniques non modifiées sont par exemple les produits vendus sous la dénomination VIDOGUM GH 175 par la société UNIPECTINE et sous la dénomination JAGUAR par la société MEYHALL.

Les gommes de guar non-ioniques modifiées utilisables selon l'invention sont de préférence modifiées par des groupements hydroxyalkyle en $C_1$-$C_6$. On peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.

Les radicaux alkyle des polymères non ioniques ont de 1 à 6 atomes de carbone sauf mention contraire.

Selon l'invention, on peut également utiliser les polymères non ioniques filmogènes dérivés d'hydroxystyrène, et notamment ceux susceptibles d'être obtenus par la copolymérisation de:

(i) au moins un monomère hydroxystyrène, l'un de ses précurseurs ou l'un de ses sels, le groupe phényl de ce monomère comprenant au moins un motif hydroxyle et étant éventuellement substitué par un ou plusieurs radicaux choisis dans le groupe comprenant les radicaux alkyles en $C_1$ à $C_{12}$, alcoxyalkyles en $C_1$ à $C_{12}$, halogènes, $SO_3H$, acyles ($C_2$ à $C_{12}$) amino, carboxy, carboxyalkyles en $C_1$ à $C_{12}$, et

(ii) au moins un autre monomère copolymérisable avec ledit monomère hydroxystyrène, l'un de ses précurseurs ou l'un de ses sels, cet autre monomère étant choisi dans le groupe comprenant les monomères acrylique, méthacrylique ou leurs dérivés, notamment les acrylates ou méthacrylates d'alkyle linéaire, cyclique ou ramifié en $C_1$ à $C_{10}$, tels que les acrylates ou méthacrylates de méthyle, d'éthyle, de propyle, de butyle, d'isobutyle, de tertiobutyle, et d'éthyl-2-hexyle, les acrylamides ou méthacrylamides N-substituées ou N,N'-substituées, les monomères de vinyle tels que les halogénures de vinyle, les esters de vinyle, notamment le benzoate de vinyle, le tertiobenzoate de vinyle ou l'acétate de vinyle.

Les monomères vinyliques, acryliques ou méthacryliques peuvent comporter un ou plusieurs groupes siloxane. Dans ce cas, ils sont choisis, en particulier, dans le groupe comprenant:

- les monomères de formule $CH_2=C(CH_3)-C-O-(CH_2)_3-Si-O-Si(CH_3)_2-CH_3$,

- les macromonomères siliconés à terminaison monofonctionnelle vinylique, allylique, ester éther ou amide de l'acide acrylique ou méthacrylique, de formule $CH_2=C(R_1)-C-X-R_2-Si(CH_3)(R_4)-O-Si(CH_3)_2-R_3$,
dans laquelle:

    - $R_1$ représente H ou $CH_3$,
    - X représente O ou NH,

- $R_2$ représente $(CH_2)_p$, p étant un entier pouvant être nul,
- $R_3$ et $R_4$ représentent, de manière indépendante, $CH_3$ ou un groupe aliphatique, cycloaliphatique ou aromatique en $C_1$ à $C_{12}$; le monomère vinylique, allylique, ester éther ou amide de l'acide acrylique ou méthacrylique comportant un ou plusieurs groupes halogénés, en particulier chlorés et/ou fluorés et/ou comportant un groupe absorbant dans l'UVA et/ou l'UVB, en particulier les groupements benzylidène camphre et benzotriazole, substitués ou non.

Les polymères du type hydroxystyrène peuvent également être obtenus par une réaction d'homopolymérisation qui peut être effectuée avantageusement à partir d'acétoxystyrène pour obtenir le poly-p-acétoxystyrène et être suivie d'une hydrolyse. Elle peut également être effectuée en milieu acide à partir du 4-hydroxyphénylméthyl carbinol.
Selon ce mode de réalisation de l'invention, le polymère est avantageusement un homopolymère appartenant à la famille des poly-p-hydroxystyrène, et de préférence on choisit le poly-4-hydroxystyrène.
Au sens de la présente invention, on entend par *"précurseur d'un monomère hydroxystyrène"* tout dérivé phénolique susceptible de conduire à l'hydroxystyrène par la voie d'une réaction de dégradation en milieu acide, notamment par déshydratation en milieu acide. A titre d'exemple, le précurseur peut être l'acétoxystyrène ou le 4-hydroxyphénylméthyl carbinol et la réaction de dégradation peut, dans ce cas, être schématisée de la façon suivante:

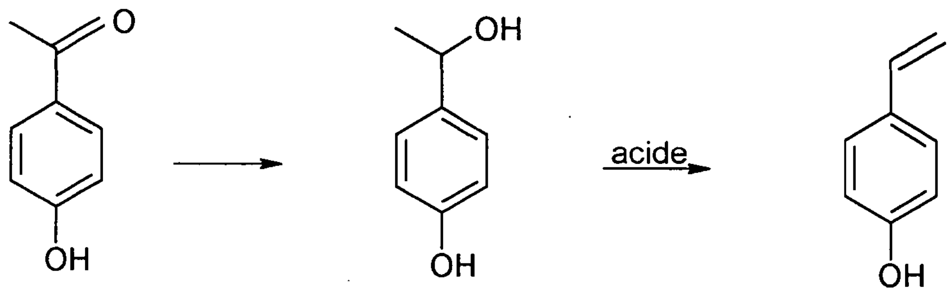


Le PHS-E est un homopolymère répondant à la formule:

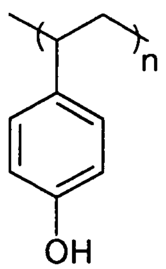

(PHS-E)

Le PHS-E est obtenu à partir de l'acétoxystyrène au moyen d'une polymérisation radicalaire suivie d'une hydrolyse. Sa masse moléculaire est comprise entre 8 000 et 100 000 g/mol. Le PHS-E est un polymère linéaire.
Le PHS-PG est un polymère répondant à la formule:

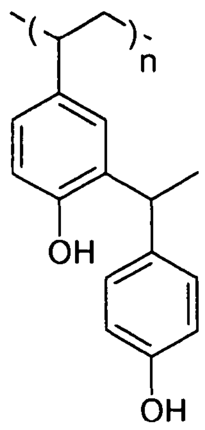

(PHS-PG)

Le PHS-PG est obtenu par polymérisation catalysée en milieu acide, à partir du 4-hydroxyphénylméthyl carbinol. Sa masse moléculaire est comprise entre 4000 et 7000 g/mol.

Le PHS-N est un polymère répondant à la formule:

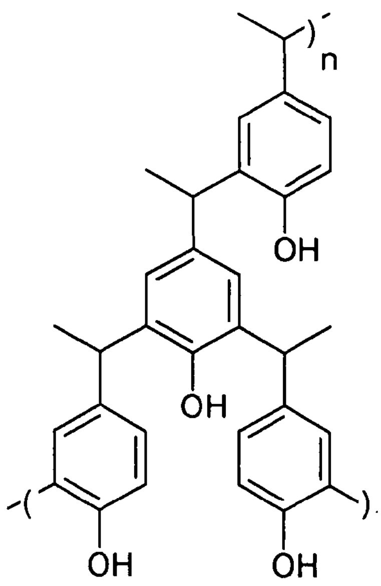

(PHS-N)

Le PHS-N est un polymère obtenu par polymérisation catalysée en milieu acide du 4-hydroxyphénylmétylcarbinol. Sa masse moléculaire est comprise entre 4 000 et 7000 g/mol.

Le PHS-PG-L est un polymère présentant la même formule brute que le PHS-E. Toutefois, le procédé d'obtention est différent puisque la polymérisation est effectuée à partir de monomères hydroxystyrène.

Dans les compositions conformes à l'invention, le ou les polymères filmogènes (A) sont, de préférence, présents à des concentrations comprises entre 0,05 et 20 % en poids, plus préférentiellement comprises entre 0,1 et 15 % en poids, et plus préférentiellement entre 0,25 et 10 % en poids par rapport au poids total de la composition.

Dans les compositions conformes à l'invention, le ou les polymères filmogènes (B) sont de préférence présents à des concentrations comprises entre 0,05 et 20 % en poids, plus préférentiellement comprises entre 0,1 et 15 % en poids, et plus préférentiellement entre 0,25 et 10 % en poids par rapport au poids total de la composition.

On choisit avantageusement les concentrations en polymères (A) et (B), de sorte que le rapport de la concentration en polymère (A) à la concentration en polymère (B) soit compris entre 4000 et 0,002.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en $C_1$-$C_4$.

Parmi ces alcools, on peut citer l'éthanol, l'isopropanol. L'éthanol est particulièrement préféré.

La composition de l'invention peut également contenir au moins un additif choisi parmi les épaississants, les tensioactifs, les parfums, les conservateurs, les filtres solaires, les protéines, les vitamines, les polymères non fixants, minérales ou synthétiques et tout autre additif classiquement utilisé dans les compositions cosmétiques destinées à être appliquées sur les cheveux.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Ces compositions peuvent être conditionnées sous diverses formes, notamment dans des flacons pompes ou dans des récipients aérosols, afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux. Les compositions conformes à l'invention peuvent aussi se présenter sous la forme de crèmes, de gels, d'émulsions, de lotions ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions conformes à l'invention peuvent être appliquées sur des cheveux secs ou humides.

L'invention va être plus complètement illustrée à l'aide de l'exemple non limitatif suivant.

Tous les pourcentages sont des pourcentages relatifs en poids par rapport au poids total de la composition et m.a. signifie matière active.

## EXEMPLE

On réalise une composition de coiffage conforme à la présente invention comprenant, comme polymère (A), de l'Avalure AC315 et, comme polymère (B), du polyvinylcaprolactame et une composition conforme à l'art antérieur comprenant, comme polymère (A), de l'Avalure AC315 et, comme polymère (B), de l'éthyl cellulose. Le tableau 1 ci-après résume les formulations réalisées.

Tableau 1

| | Composition 1 (art antérieur) | Composition 2 (invention) |
|---|---|---|
| Avalure AC315 | 1,5 % | 2,65 % |
| Ethyl cellulose | 0,2 % | ---- |
| polyvinylcaprolactame | --- | 0,35 % |
| Ethanol | q.s 100 | q.s. 100 |

On applique les deux compositions conditionnées en aérosol (composition 65 %, DME 35 %) sur des mèches de cheveux eurochâtains de longueur 18 cm et de 20 grammes.

On évalue la douceur et la facilité de démêlage des cheveux après application de ces compositions sur les cheveux au moyen d'un test sensoriel avec un panel de 5 personnes. Les notes attribuées vont de 0 (mauvaise performance) à 50 (excellente performance). Le démêlage évalué est la facilité de repassage du peigne effectué après un premier démêlage destiné à rompre les structures polymère-cheveu.

Les résultats obtenus sont résumés dans le tableau 2 ci-après.

Tableau 2

| | Composition 1 (art antérieur) | Composition 2 (invention) |
|---|---|---|
| douceur | 10 | 20 |
| démêlage | 20 | 25 |

Il en résulte que les compositions conformes à l'invention procurent de meilleurs résultats en terme douceur et de démêlage que les compositions conformes à l'art antérieur.

## Revendications

1. Composition de coiffage comprenant, dans un milieu cosmétiquement acceptable:

   (1) au moins un polymère (A) choisi de telle sorte qu'un film obtenu par séchage d'un mélange de ce polymère (A) avec de l'éthanol ou de l'eau, à température ambiante et à un taux d'humidité relative de 50 %, présente un profil mécanique défini par au moins:

   (i) un taux d'allongement à la rupture ($\varepsilon_r$) supérieur ou égal à 300 %;
   (ii) une recouvrance à 300 secondes ($R_{300}$) supérieure ou égale à 45 %; et
   (iii) lorsque la recouvrance à 300 secondes est comprise entre 45 et 60 %, alors l'allongement est inférieur à 1300 %;

   (2) au moins un polymère (B) filmogène, non ionique, différent du polymère (A) et choisi parmi

   - les polyalkyloxazolines;
   - les homopolymères d'acétate de vinyle;
   - les copolymères d'acétate de vinyle et d'ester acrylique;
   - les copolymères d'acétate de vinyle et d'éthylène;
   - les copolymères d'acétate de vinyle et d'ester maléïque;
   - les copolymères de polyéthylène et d'anhydride maléïque;
   - les homopolymères d'acrylates d'alkyle et les homopolymères de méthacrylates d'alkyle;
   - les copolymères d'esters acryliques tels que par exemple les copolymères d'acrylates d'alkyle et de méthacrylates d'alkyle ;
   - les copolymères d'acrylonitrile et d'un monomère non ionique choisi parmi le butadiène et les (méth)acrylates d'alkyle,
   - les copolymères d'acrylate d'alkyle et d'uréthanne,
   - les gommes de guar,
   - les polymères dérivés d'hydroxystyrène, et
   - les homo ou copolymères de vinylactames.

2. Composition selon la revendication 1, **caractérisée par le fait que** les polymères (B) dérivés d'hydroxystyrène sont des polymères linéaires ou branchés susceptibles d'être obtenus par un procédé comprenant une homo- ou une (co)-polymérisation d'au moins un monomère hydroxystyrène, l'un de ses précurseurs ou l'un de ses sels, le groupe phényl de ce monomère comprenant au moins un motif hydroxyle et étant éventuellement substitué par un ou plusieurs radicaux choisis dans le groupe comprenant les radicaux alkyles en $C_1$ à $C_{12}$, alcoxyalkyles en $C_1$ à $C_{12}$, halogènes, $SO_3H$, acyles ($C_2$ à $C_{12}$) amino, carboxy, carboxyalkyles en $C_1$ à $C_{12}$.

3. Composition selon la revendication 2, **caractérisée, par le fait que** le polymère (B) filmogène est susceptible d'être obtenu par la copolymérisation de:

   (i) au moins un monomère hydroxystyrène, l'un de ses précurseurs ou l'un de ses sels, le groupe phényl de ce monomère comprenant au moins un motif hydroxyle et étant éventuellement substitué par un ou plusieurs radicaux choisis dans le groupe comprenant les radicaux alkyles en $C_1$ à $C_{12}$, alcoxyalkyles en $C_1$ à $C_{12}$, halogènes, $SO_3H$, acyles ($C_2$ à $C_{12}$) amino, carboxy, carboxyalkyles en $C_1$ à $C_{12}$, et
   (ii) au moins un autre monomère copolymérisable avec ledit monomère hydroxystyrène, l'un de ses précurseurs ou l'un de ses sels, cet autre monomère étant choisi dans le groupe comprenant les monomères acrylique, méthacrylique ou leurs dérivés, notamment les acrylates ou méthacrylates d'alkyle linéaires, cycliques ou ramifiés en $C_1$ à $C_{10}$, les acrylamides ou méthacrylamides N-substituées ou N,N'-substituées, les monomères de vinyle tels que les halogénures de vinyle, les esters de vinyle, notamment le benzoate de vinyle, le tertiobenzoate de vinyle ou l'acétate de vinyle.

4. Composition selon l'une quelconque des revendications précédentes, **caractérisée par le fait que** le ou les polymères filmogènes (A) sont présents à des concentrations comprises entre 0,05 et 20 % en poids, plus préférentiellement comprises entre 0,1 et 15 % en poids, et plus préférentiellement entre 0,25 et 10 % en poids par rapport au poids total de la composition.

**5.** Composition selon l'une quelconque des revendications précédentes, **caractérisée par le fait que** le ou les polymères filmogènes (B) sont présents à des concentrations comprises entre 0,05 et 20 % en poids, plus préférentiellement comprises entre 0,1 et 15 % en poids, et plus préférentiellement entre 0,25 et 10 % en poids par rapport au poids total de la composition.

**6.** Composition selon l'une quelconque des revendications précédentes, **caractérisée par le fait que** le rapport de la concentration en polymère (A) à la concentration en polymère (B) soit compris entre 4000 et 0,002.

**7.** Composition selon l'une quelconque des revendications précédentes, **caractérisée par le fait qu'**elle contient en outre des additifs cosmétiques conventionnels choisis dans le groupe comprenant les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les protéines et les vitamines.

**8.** Dispositif aérosol formé par un récipient contenant une composition selon l'une quelconque des revendications précédentes et un gaz propulseur, ainsi qu'un moyen de distribution de la composition.

**9.** Procédé de maintien ou de mise en forme de la coiffure, **caractérisé par le fait qu'**il comprend la mise en oeuvre d'une composition conforme à l'une quelconque des revendications 1 à 7.

**10.** Utilisation d'une composition selon l'une quelconque des revendications 1 à 7, pour la fabrication d'un produit cosmétique capillaire, en vue de maintenir et/ou de fixer la coiffure.

Office européen des brevets

# RAPPORT PARTIEL DE RECHERCHE EUROPEENNE

qui selon la règle 45 de la Convention sur le brevet européen est consideré, aux fins de la procédure ultérieure, comme le rapport de la recherche européenne

Numéro de la demande
EP 05 29 1946

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | WO 97/15275 A (PROCTER & GAMBLE) 1 mai 1997 (1997-05-01) * revendication 1 * ----- | 1-10 | A61K7/06 |
| A | DE 43 16 242 A (HENKEL KGAA) 17 novembre 1994 (1994-11-17) * revendication 1; exemple 6 * ----- | 1-10 | |
| A | FR 2 750 047 A (OREAL) 26 décembre 1997 (1997-12-26) * revendications 1-16; exemples 1,3 * ----- | 1-10 | |
| A | EP 0 265 228 A (SHIONOGI & CO ;NISSHIN CHEMICALS CO (JP)) 27 avril 1988 (1988-04-27) * page 10, ligne 20 - ligne 30; revendications * ----- | 1 | |
| A | PATENT ABSTRACTS OF JAPAN vol. 096, no. 007, 31 juillet 1996 (1996-07-31) & JP 08 081349 A (PENTEL KK), 26 mars 1996 (1996-03-26) * abrégé * ----- | 1 | DOMAINES TECHNIQUES RECHERCHES (IPC) |
| | -/-- | | A61K |

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| La Haye | 7 décembre 2005 | Loiselet-Taisne, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C08)

4

Office européen des brevets

**RAPPORT PARTIEL DE RECHERCHE EUROPEENNE**

Numero de la demande
EP 05 29 1946

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| P,A | "Avalure Film Forming Polymers for Personal Care Applications" BFGOODRICH, [Online] XP002109281 Extrait de l'Internet: URL:http://www.carbopol.com/techdata/pdf/tds248.pdf> [extrait le 1999-07-14] * page 3 * | 1 | |
| A | EP 0 605 951 A (ADVANCED GENETIC TECH) 13 juillet 1994 (1994-07-13) * revendications * | 1-3 | |
| | | | DOMAINES TECHNIQUES RECHERCHES (IPC) |

4

EPO FORM 1503 03.82 (P04C11)

Office européen des brevets

**RECHERCHE INCOMPLETE FEUILLE SUPPLEMENTAIRE C**

Numéro de la demande
EP 05 29 1946

Revendications ayant fait l'objet de recherches incomplètes:
1-10

Revendications n'ayant pas fait l'objet de recherches:
-

Raison pour la limitation de la recherche:

Les revendications 1-10 présentes ont trait à une composition/un dispositif/un procédé/une utilisation défini(e) (entre autres) en ce qu'elle/il comprend au moins un polymère (A) défini

au moyen des paramètres suivants:

le profil mécanique du film obtenu par séchage d'un mélange de ce polymère (A) avec de l'éthanol ou de l'eau, à température ambiante et à un taux d'humidité relative de 50% défini par au moins:
(i) un taux d'allongement à la rupture supérieur ou égal à 300%;
(ii) une recouvrance à 300 secondes supérieure ou égale à 45%; et
(iii) lorsque la recouvrance à 300 secondes est comprise entre 45 et 60%, alors l'allongement est inférieur à 1300%.

L'utilisation de ces paramètres est considérée , dans le présent contexte, comme menant à un manque de clarté au sens de l'Article 84 CBE. Il est impossible de comparer les paramètres que le déposant a choisi d'utiliser avec ce qui est révélé dans l'état de la technique. Le manque de clarté qui en découle est tel q'une recherche significative complète est impossible. Par conséquent, la recherche a été limitée à une composition/un dispositif/un procédé/une utilisation de coiffage comprenant dans un milieu cosmétiquement acceptable comprenant: (1) au moins un polymère filmogène acrylique (A) et (2) au moins un polymère (B) tel que défini à la revendication 1. La définition de (A) sur laquelle est basée la recherche couvre en partie celle telle que revendiqué. Cette limitation est basée sur la description à la page 3 lignes 27,28 et sur l'unique exemple.

## ANNEXE AU RAPPORT DE RECHERCHE EUROPEENNE RELATIF A LA DEMANDE DE BREVET EUROPEEN NO.

EP 05 29 1946

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-12-2005

| Document brevet cité au rapport de recherche | | Date de publication | Membre(s) de la famille de brevet(s) | | | Date de publication |
|---|---|---|---|---|---|---|
| WO 9715275 | A | 01-05-1997 | AT | 235213 | T | 15-04-2003 |
| | | | AU | 7455896 | A | 15-05-1997 |
| | | | BR | 9611585 | A | 13-07-1999 |
| | | | CA | 2235813 | A1 | 01-05-1997 |
| | | | CN | 1200664 | A | 02-12-1998 |
| | | | DE | 69627002 | D1 | 30-04-2003 |
| | | | DE | 69627002 | T2 | 11-03-2004 |
| | | | EP | 0861064 | A1 | 02-09-1998 |
| | | | HK | 1015700 | A1 | 21-01-2004 |
| | | | JP | 10512294 | T | 24-11-1998 |
| | | | JP | 3088468 | B2 | 18-09-2000 |
| | | | US | 6248316 | B1 | 19-06-2001 |
| DE 4316242 | A | 17-11-1994 | WO | 9426236 | A1 | 24-11-1994 |
| FR 2750047 | A | 26-12-1997 | AUCUN | | | |
| EP 0265228 | A | 27-04-1988 | AU | 602731 | B2 | 25-10-1990 |
| | | | AU | 8008887 | A | 28-04-1988 |
| | | | CA | 1299319 | C | 21-04-1992 |
| | | | DE | 3779250 | D1 | 25-06-1992 |
| | | | ES | 2031909 | T3 | 01-01-1993 |
| | | | GR | 3004890 | T3 | 28-04-1993 |
| | | | KR | 9511449 | B1 | 04-10-1995 |
| | | | NZ | 222096 | A | 21-12-1990 |
| | | | US | 4874830 | A | 17-10-1989 |
| | | | US | 4914140 | A | 03-04-1990 |
| JP 08081349 | A | 26-03-1996 | AUCUN | | | |
| EP 0605951 | A | 13-07-1994 | AT | 181659 | T | 15-07-1999 |
| | | | DE | 69325501 | D1 | 05-08-1999 |
| | | | DE | 69325501 | T2 | 25-11-1999 |
| | | | JP | 7309720 | A | 28-11-1995 |
| | | | US | 5547660 | A | 20-08-1996 |

EPO FORM P0460

Pour tout renseignement concernant cette annexe : voir Journal Officiel de l'Office européen des brevets, No.12/82